# EUROPEAN PATENT SPECIFICATION

(11) **EP 0 737 450 B1**
(45) Date of publication and mention of the grant of the patent: **12.11.2003**
(21) Application number: 96200976.7
(22) Date of filing: 09.04.1996
(51) Int. Cl.: A61F 2/01, A61B 17/22

(54) **Catheter with filter and thrombidischarge device**
Katheter mit Filter und Vorrichtung zur Abfuhr von Thromben
Cathéter à filtre et dispositif pour l'évacuation des thrombis

(30) Priority: 10.04.1995 NL 1000105
(43) Date of publication of application: 16.10.1996
(73) Proprietor: CORDIS EUROPA N.V., NL-9301 LJ Roden (NL)
(72) Inventor: Boudewijn, Alexander Christiaan, 9351 KS Leek (NL)
(74) Representative: 't Jong, Bastiaan Jacob

(56) References cited:
- EP-A- 0 442 579
- FR-A- 2 606 642
- US-A- 4 723 549
- US-A- 5 053 008

## Description

The invention relates to a catheter comprising a tube-like basic body with a distal and a proximal end and wherein a filter element has been arranged to the distal end.

A catheter comprising a tube-like body with a filter element and a device for reducing and removing thrombi is introduced into a patient. At the distal end the filter element is formed by a number of strip-shaped wall sections arranged longitudinally along the basic body. The filter element is activated by means for moving sections of the basic body situated on either side of said strip-shaped wall sections towards each other, which bend the sections outwards. Such a catheter is known from FR-A-2 606 642.

The object of the invention is to provide a catheter of the type as described in the preamble of claim 1 with which removal of the collected particles is accelerated.

This aim is achieved with the catheter according to the invention as characterized in claim 1. As a result the thrombi collected by the filter element can be fragmented and removed from the body. A stream of liquid under pressure, supplied via the pressure lumen, flows out of the jet nozzle in the form of a jet and engages sections of thrombi which are fragmented due to the energy of the jet. The thrombus fragments are conveyed by the jet to the discharge opening and removed via the discharge lumen. This can be done at regular intervals or just before the catheter is removed from the body of the patient.

The reduction and removal device is arranged within the filter element and can therefor continue to work properly without getting blocked. On activating the reduction and removal device, first those parts of the thrombi extending as far as in between the bent wall sections of the filter element are engaged, after which gradually the entire structure is fragmented and removed.

The wall sections defined in between the longitudinal cuts can bend outwards until they make contact with the wall of the blood vessel inside of which the filter element is placed. The thrombi arrested by the filter element are sucked into and remain enclosed within the filter element until these are removed. Prior to introducing the catheter, the wall sections are straightened by moving the ends of the basic body situated on either side of the wall sections away from each other. As a result the catheter will obtain a small cross-section equal to that of the basic body not provided with the longitudinal cuts, so that the catheter can be introduced with a minimum of traumatic effects.

A very suitable embodiment of the catheter according to the invention is characterised in claim 2. By moving the inner and outer tube-like bodies in relation to one another the strip-shaped wall sections can be bent outwards or stretched respectively. On introducing the catheter, the inner tube-like body is moved as much as possible in the relatively distal direction inside the outer tube-like body, so that the strip-shaped wall sections will be stretched. After positioning the distal end of the catheter, the inner tube-like body is moved in the relatively proximal direction in the outer tube-like body, as a result of which the strip-shaped wall sections will bend outwards and consequently form the filter element.

Additionally the measure as set out in claim 3 is preferably employed. The inner tube-like body is in that case essentially made in the form of a suction catheter, wherein all parts required for the reduction and removal device are received in this inner tube-like body.

Preferably the measure as set out in claim 4 is employed. The discharge lumen does not need to be connected in that case to a separate source of suction. As a result of the ejector action a sufficient flow is maintained in the discharge lumen without applying additional suction.

It is noted that another catheter is known from US-A-5 053 008, which is introduced into a patient during surgery in order to collect thrombi and to prevent that these finish up for instance in the vascular system of the lungs and cause an embolism. The filter element of such a catheter is usually positioned in the vena cava. The collected particles are removed by disintegrating them locally. Removal of the particles by allowing them to disintegrate locally can take up quite some time.

Figure 1 shows schematically the application of a catheter according to the invention.

Figure 2 illustrates the distal end of a catheter according to the invention when being positioned in the blood vessel.

Figure 3 shows a view corresponding to that of figure 2 in which case the filter element is illustrated in working state.

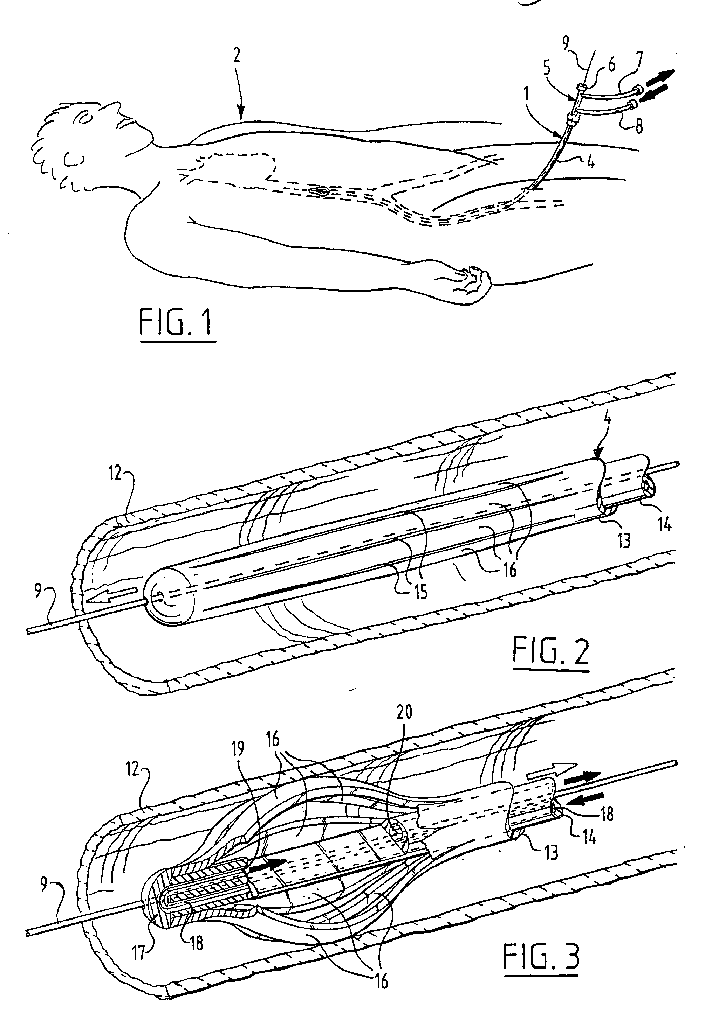

As is illustrated schematically in figure 1, a catheter 1 according to the invention is introduced into a patient 2, in order to position a filter element 3 at the distal end of the catheter 1 inside a blood vessel, in particular in the vena cava.

The catheter 1 comprises a tube-like basic body 4 with a filter element 3, still to be explained in greater detail, at the distal end and a connecting member 5 at the proximal end.

The connecting member 5 comprises in this example of an embodiment a haemostatic valve 6 through which a guide wire 9 is advanced, which is employed in the usual manner for the purpose of positioning the catheter 1.

Furthermore the connecting member 5 comprises a discharge connection 7 which is connected with the discharge lumen in the basic body 4 and a pressure connection 8 which is connected with a pressure lumen in the basic body 4.

The basic body 4 of the catheter 1 comprises an outer tube-like body 13 and an inner tube-like body 14 received therein in a, in a longitudinal direction, movable manner. The outer tube-like body 13 and the inner tube-like body 14 are connected to each other with their distal ends. At the very end, a tip 17, made of a soft material, has been arranged in order to prevent trauma on introduction of the catheter 1 into the blood vessel 12.

At the distal end of the outer tube-like body 13, a number of longitudinal cuts 15, distributed evenly around the circumference, have been arranged defining strip-shaped wall sec- tions 16 in between them. The wall sections 16 together form the actual filter element. By pulling the inner tube-like body 14 at the proximal end of the catheter 1 over a limited distance in relation to the outer tube-like body 13 outwards, the strip-shaped wall sections are put under a compression load in a longitudinal direction as a result of which they will bend outwards in the direction illustrated in figure 3. The relative displacement of the outer tube-like body and the inner tube-like body is chosen in such a manner, that the wall sections 16 make a good contact with the inner wall of the blood vessel 12.

It will be clear that thrombi as from a certain size, flowing through the blood vessel 12, will be collected by the filter element formed by the wall sections 16 bending outwards, and that at the same time the blood can flow in a normal manner through these wall sections.

The inner tube-like body 14 comprises the aforementioned pressure lumen 18 connected with the pressure connection 8. In the manner illustrated in figure 3, the pressure lumen 18 is connected with a channel section extending backwards in proximal direction forming a jet nozzle 19. The jet nozzle 19 is directed at a discharge opening 20 which forms the end section of a discharge lumen in the inner tube-like body 14, which is connected in the above described manner with the discharge connection 7.

In addition to the pressure lumen and the discharge lumen, the inner tube-like body 14 comprises another lumen for the guide wire 9.

End sections of thrombi collected by the filter element will extend as far as inside the space defined by the wall sections 16 bending outwards. In order to reduce the thrombi in size and remove them, liquid under pressure is supplied to the pressure connection 8, so that a powerful liquid jet leaves the pressure nozzle 19 in the direction of the discharge opening 20. The end sections of the thrombi are engaged by this liquid jet and reduced in size by mechanical action and entrained through the discharge opening 20 in the discharge lumen. In order to stimulate the discharge flow, a vacuum source can be connected to the discharge connection 7. As a result of the suction action, the thrombi are gradually sucked further into the space defined by the wall sections 16 where they are gradually reduced in size and ultimately removed via the discharge lumen.

The jet nozzle 19 and the discharge opening 20 can, in a suitable manner, be arranged and dimensioned in relation to each other in such a way that the flow of liquid leaving the jet nozzle 19 creates an ejector-action as a result of which a separate flow is created in the discharge lumen towards the proximal end without additional suction action at the discharge connection 7.

It will be clear that the thrombi reduction and discharge device, and the filter element likewise, can be embodied in different ways. The reduction and discharge device may for instance comprise several, for instance two, jet nozzles with corresponding discharge openings. It will also be possible to arrange the reduction and discharge device at for instance the relatively distal end of the filter element, that is to say outside the space defined by the wall sections bending outwards. Another possibility is a combination of a reduction and discharge device both in and outside the filter element.

When the filter catheter has carried out its task, and there is no longer a danger for the patient of an embolism forming, the catheter can be removed. In order to achieve this, the inner tube-like body is moved in relation to the outer tube-like body in a relatively distal direction, so that the wall sections 16 bending outwards are stretched again into the state illustrated in figure 2. The catheter will then have resumed its original small cross-section, so that it can be withdrawn easily.

## Claims

1. Catheter (1) comprising a tube-like basic body (4) with a distal and a proximal end, wherein the catheter comprises at the distal end a filter element (3) formed by a number of strip-shaped wall sections (16) of the basic body (4) defined in between longitudinal cuts (15) evenly distributed around the circumference, a device for reducing thrombi in size and removing them (19,20), and means for moving sections of the basic body situated on either side of the wall sections (16) towards each other in order to make the wall sections bend outwards **characterised in that** the thrombi reduction and discharge device comprises a jet nozzle (19) connected with a pressure lumen (18) inside the basic body (4) pointing in the proximal direction and a discharge opening (20), formed opposite the jet nozzle (19), which is connected to a discharge lumen inside the basic body (4), and **in that** the jet nozzle (19) and the discharge opening (20) are positioned inside the longitudinal section of the catheter (1) wherein the longitudinal cuts (15) extend.

2. Catheter as claimed in claim 1, comprising an outer tube-like body (13) and an inner tube-like body (14) received inside it in a movable manner, which are connected to each other at their distal ends (17) and wherein the strip-shaped wall sections (16) have been formed in the outer tube-like body (13).

3. Catheter as claimed in claim 1 and 2, wherein the pressure lumen (18) and the discharge lumen have been received inside the inner tube-like body (14).

4. Catheter as claimed in one of the preceding claims, wherein the jet nozzle (19) has been positioned and dimensioned in relation to the discharge opening (20) in such a way that they form an ejector.

## Patentansprüche

1. Katheter (1), aufweisend einen rohrartigen Basiskörper (4) mit einem distalen und einem proximalen Ende, wobei der Katheter an dem distalen Ende ein Filterelement (3) aufweist, das von einer Anzahl von streifenförmigen Wandabschnitten (16) des Basiskörpers (4) gebildet wird, die zwischen Längseinschnitten (15) definiert sind, die um den Umfang gleichmäßig verteilt sind, eine Vorrichtung zum Reduzieren von Thromben in der Größe und deren Entfernen (19, 20), und Mittel zum Bewegen von Abschnitten des Basiskörpers, die an jeder Seite der Wandabschnitte (16) aufeinander zu angeordnet sind, um die Wandabschnitte nach außen biegbar zu machen, **dadurch gekennzeichnet, dass** die Vorrichtung zum Reduzieren und Auslassen von Thromben eine Strahldüse (19), die mit einem Drucklumen (18) innerhalb des Basiskörpers (4) in die proximale Richtung weisend verbunden ist, und eine der Strahldüse (19) gegenüberliegend ausgebildete Auslassöffnung (20) aufweist, welche mit einem Auslasslumen innerhalb des Basiskörpers (4) verbunden ist, und dass die Strahldüse (19) und die Auslassöffnung (20) innerhalb des Längsabschnitts des Katheters (1), in dem sich die Längseinschnitte (15) erstrecken, positioniert sind.

2. Katheter nach Anspruch 1, aufweisend einen äußeren rohrartigen Körper (13) und einen inneren rohrartigen Körper (14), die in diesem in einer beweglichen Weise aufgenommen sind, welche an ihren distalen Enden (17) miteinander verbunden sind, und wobei die streifenförmigen Wandabschnitte (16) in dem äußeren rohrartigen Körper (13) gebildet wurden.

3. Katheter nach Anspruch 1 und 2, wobei das Drucklumen (18) und das Auslasslumen innerhalb des inneren rohrartigen Körpers (14) aufgenommen wurden.

4. Katheter nach einem der vorhergehenden Ansprüche, wobei die Strahldüse (19) relativ zu der Auslassöffnung (20) in einer solchen Weise positioniert und dimensioniert wurden, dass sie einen Ejektor bilden.

## Revendications

1. Cathéter (1) comprenant un corps (4) de base en forme de tube avec une extrémité distale et une extrémité proximale, dans lequel le cathéter comprend à l'extrémité distale un élément (3) de filtre formé par un certain nombre de sections (16) de paroi en forme de bande du corps (4) de base définies entre des découpes longitudinales (15) réparties uniformément autour de la circonférence, un dispositif (19, 20) pour réduire des thrombus en taille et les évacuer, et des moyens pour déplacer les unes vers les autres des sections du corps de base situées sur l'un ou l'autre côté des sections (16) de paroi afin d"amener les sections de paroi à s'incurver vers l'extérieur, **caractérisé en ce que** le dispositif de réduction et d'évacuation de thrombus comprend une buse (19) d'éjection reliée à une lumière (18) de pression à l'intérieur du corps (4) de base orientée dans la direction proximale, et une ouverture (20) d'évacuation, formée à l'opposé de la buse (19) d'éjection, qui est reliée à une lumière d'évacuation à l'intérieur du corps (4) de base, et **en ce que** la buse (19) d'éjection et l'ouverture (20) d'évacuation sont positionnées à l'intérieur de la section longitudinale du cathéter (1) dans lequel s'étendent les découpes longitudinales (15).

2. Cathéter selon la revendication 1, comprenant un corps extérieur (13) en forme de tube et un corps intérieur (14) en forme de tube logé de manière mobile à l'intérieur de celui-ci, qui sont reliés l'un à l'autre à leurs extrémités distales (17), et dans lequel les sections (16) de paroi en forme de bande ont été formées dans le corps extérieur (13) en forme de tube.

3. Cathéter selon la revendication 1 ou 2, dans lequel la lumière (18) de pression et la lumière d'évacuation ont été reçues à l'intérieur du corps intérieur (14) en forme de tube.

4. Cathéter selon l'une des revendications précédentes, dans lequel la buse (19) d'éjection a été positionnée et dimensionnée par rapport à l'ouverture (20) d'évacuation de manière qu'elles forment un éjecteur.
